# EUROPEAN PATENT SPECIFICATION

(11) **EP 1 706 852 B1**
(45) Date of publication and mention of the grant of the patent: **13.08.2008**
(21) Application number: 05701817.8
(22) Date of filing: 10.01.2005
(51) Int. Cl.: G07F 7/10, G07C 9/00

(54) **AN IDENTIFICATION CARD AND A METHOD OF IDENTIFYING A CARD HOLDER USING THE CARD**
IDENTIFIKATIONSKARTE UND VERFAHREN ZUM IDENTIFIZIEREN EINES KARTENHALTERS MIT DER KARTE
CARTE D'IDENTIFICATION ET PROCEDE D'IDENTIFICATION DE PORTEUR DE CARTE AU MOYEN DE CETTE CARTE

(30) Priority: 09.01.2004 GB 0400428
(43) Date of publication of application: 04.10.2006
(73) Proprietor: Kinderguard Limited, Holywood BT18 9HF (GB)
(72) Inventor: DOUGLAS, Raymond, Newcastle, County Down BT33 0PP (GB)
(74) Representative: McCarthy, Denis Alexis
(86) International application number: PCT/GB2005/000047
(87) International publication number: WO 2005/066906

(56) References cited:
- WO-A-01/20538
- WO-A-01/52180

## Description

The present invention relates to an identification card and a method of identifying a card holder using the card and in particular to an identification card using biometric characteristics for identification. The term card holder refers to a person who holds the card between their index finger and thumb under normal operating conditions. However, it will of course be appreciated that a physically compromised person may hold the card between any of their adjacent body parts, such as fingers, toes allowing operation of the invention.

Biometrics is well researched, and there seems to be virtually no limit to the body parts, personal characteristics and reading methods used for biometric identification: fingers, hands, feet, faces, eyes, ears, teeth, veins, voices, signatures, typing styles, Human DNA, gaits and odours. Biometrics tackles the weakest links in our systems, and works on improving the security of those elements to mitigate risk against security attacks, of which on average eighty percent are password-related.

A snapshot of where biometric applications are being used shows that physical access control i.e. protecting buildings and rooms from unauthorized entry is credited with roughly half of all sales of biometric devices in the year 2001. Logical access control i.e. protecting computers and networks represented forty percent of the market and time and attendance applications accounted for the remainder. There are many biometric technologies that are readily available for exploitation. The existing key biometric applications currently developed and available are summarised as follows:

Hand Geometry - The hand is very robust and would require considerable physical damage to significantly alter the geometry. The hand is not very distinctive, with many people sharing common geometrical representation. The hand is accessible as it is easy to present The hand is acceptable as opinion is that it does not invade personal privacy. The hand availability is low as typically only one hand geometry can be presented, as the left hand is often a near mirror image of the right.

Fingerprints - The fingerprint is not very robust and is susceptible to chemical and physical damage. The fingerprint is very distinctive with primary applications based on criminal identification within the Law Enforcement agencies. The fingerprint is accessible but often requires real-time feedback to the user regarding proper presentation and can be complicated by skin ailments. The fingerprint is acceptable as opinion is that it does not invade personal privacy. The fingerprint availability is high as typically a person can present at least six nearly independent fingerprints. The palm can also be included within this category but is rarely used.

State-of-the-art in this area is the result of an EU funded Cost-Sharing project (IST-2000-25168) which ended in June 2002, co-ordinated by Infineon Technologies AG (DE), entitled "Biometric Matching and Authentication System on Card (Finger_Card)".

Face - The face is robust being an area of the body that is well protected. The face is not very distinctive with primary applications based on criminal identification matching to known suspects. The face is accessible as systems can capture images from distance (several meters) using video equipment but orientation problems distort the data collection. The face is acceptable as opinion is that it does not invade personal privacy. The face availability is high as typically a person can present several independent features but orientation may distort these. There is also the technical infrastructural cost associated with acquiring the source data from the face and other parts of the human body not to mention the need for the human to participate in the acquisition of such data by standing in the correct position or interacting with certain devices to source the data.

Eyes - The eye is very robust being an area of the body that is well protected. The eye is very distinctive with primary applications based on retinal scanning although iris scanning is also developed. The eye is accessible but can be complicated although retinal scanning reduces the orientation problems because the eye naturally aligns itself as it focuses on an illuminated target. Iris scanning does not require the person to interact with a device but instead uses a video image of the eye which can be taken from one foot away. The eye is acceptable as opinion is that it does not invade personal privacy. The eye availability is complicated by the fact that multiple areas of the retina can be presented by moving the eye in various directions.

Voice - The voice is robust but there are a whole range of things that impact voice verification such as colds and flu, medication and stress. The voice is very distinctive demonstrating a 0.3% false acceptance rate (FAR). The voice is accessible but does vary due to the positional changes of the body, the physical well-being of the speaker and time. The voice is acceptable and is clearly non-invasive but may not always be appropriate for some personal transactions or social environments. The voice availability is high as it is determined by many different factors: size of the vocal cavities (throat, oral, nasal) as well as the characteristics of the vocal chords themselves and is further modified by the way you speak - the way your mouth, lips, tongue, jaw and teeth move.

Skin Chemistry - The skin chemistry is not well documented with main references to fingerprinting and the authentication of a 'real' finger. The main application being researched is through the IBM patent (referenced below) which is developing DNA sampling which is known to be robust, distinctive, accessible and available.

In the area of development of a biometric sensor on-card, US Patent No. 2002095587 in the name of IBM, entitled "Smart card with integrated biometric sensor" focuses on the sampling of a fingerprint, a palm print, a voice print, a retina and/or skin chemistry.

US Patent No. 6347040 in the name of Infineon Technologies AG (DE), entitled "Sensor device for sensing biometric characteristics, in particular finger minutiae" discloses a sensor device with a biometric sensor chip. The sensor chip is fastened on a flexible printed circuit board that has a highly flexible substrate layer and conductor tracks applied to the substrate layer. The conductor tracks are in electrical contact with the sensor chip and are led to a terminal region of the flexible printed circuit board. The sensing area of the sensor chip is accessible through an opening in the flexible circuit board and the opening is at least partially surrounded by a grounding frame.

German Patent No. DE10126839 filed by Infineon Technologies AG (DE), entitled "Fingerprint sensor for personal identification incorporates electrostatic discharge protection and verification that applied finger is living tissue" focuses on the sampling of a finger to identify the presence of living tissue by diversion of a received electrostatic charge that is used for impedance measurement in association with a measuring circuit. The surface resistance is determined mainly by conductivity of a liquid (sweat) covering the skin and this technology attempts to tackle the issue of fake generated prints.

The main problem associated with the implementation of newly developed biometric 'smart cards' is a financial obstacle because of the requirement to install software and hardware at each reading facility. This provides no problem where newly developed card readers are being installed for the first time but for old ATM machines for example, the financial cost of installing hardware and software at all ATM's is prohibitive in the extreme. This problem arises in the first place because traditional biometric measurement can not be economically miniaturised sufficiently to fit on-card meaning additional external readers are required forcing the market to standardise on one biometric.

WO 01/20538 discloses a method and apparatus for authenticating an individual living organism by recognizing a unique internal electric and/or magnetic and/or acoustic characteristic, which comprises a biometric signature, by presenting a body part to a sensing device that senses the signature. The sensed presented biometric signature is compared to a known biometric signature to authenticate the individual. This authentication can then be used to authorize an action by the individual, such as accessing equipment or an area, or conducting perform actions, such as conducting such as conducting financial transactions. A card having sensors is used to sense the biometric signature which is read by a card reader and sent to a local or remote reader for comparison.

WO 01/52180 discloses a biometrically activated device wherein data derived from detection of unique internal biometric traits are stored within said biometrically activated device to be compared to stored data relative to said internal biometric markers or traits previously scanned from a user of said biometrically activated device. The biometrically activated device may allow or disallow access to information, or provide use of, data or a device protected by the biometrically activated device.

Clearly, there is a need for a new method of human identification using an identification card which is interoperable with existing systems thereby overcoming the infrastructural problem. Such a system should not require additional training on behalf of the user and should essentially provide a method of uniquely identifying the human.

Accordingly, the present invention provides an identification card having means for generating and transmitting signals into the body of a card holder and means for receiving and interpreting the signals from the card holder's body, the signals being attenuated by bioelectrical impedance of the card holder's body such that the interpretation of the attenuated signals by the interpretation means provides a bioelectrical impedance signature for uniquely identifying the card holder, wherein the means for generating and transmitting signals into the body of a card holder and the means for receiving and interpreting the signals from the card holder's body comprises a controller having a central processing unit (C.P.U.) and an associated memory, two or more electrodes disposed on an external surface of the identification card and electronic control circuitry electrically coupling the electrodes to the controller, wherein the identification card has its own on board power source capable of providing sufficient power for independent operation of the identification card **characterised in that** the two or more electrodes are provided on opposing main faces of the identification card and are located so that the forefinger and the thumb are naturally located thereon when the card is held between the forefinger and the thumb, and the card's own power source is initiated when the card holder holds the electrodes between their finger and thumb for a predetermined period of time.

Bioelectrical impedance is measurable with simple technical instruments which facilitates the ability to miniaturise the biometric sensing technology to a degree suitable for on-card implementation.

Preferably, the electronic control circuitry comprises means for generating a range of analogue signals of varying currents and frequency.

Ideally, the currents are in the range of 100µA to 900µA.

Preferably, the frequency of the signals is in the range of 1 KHZ to 1350 KHZ.

Ideally, the electronic control circuitry has means for filtering the attenuated signals, converting the analogue signals to digital signals and passing the signals to the C.P.U. for interpretation.

Preferably, the means for generating and interpreting signals comprises a software control module stored on the memory of the controller.

Ideally, the software control module compares a live bioelectrical impedance signature with a bioelectrical impedance signature recorded in the memory of the controller. Live biometric data describes information obtained by the card from the person currently holding the card as opposed to information from the person whose biometric data was originally recorded by the card issuing authority. Such comparison is performed under the control of a neural network module that will observe, learn and verify the thresholds of the biometric data within acceptable tolerance limits.

Preferably, the software control module has means for identifying bioelectrical impedance characteristics representative of fat mass, body cell mass, extra cellular water and skeletal mass.

Ideally, the software control module is capable of generating, transmitting, receiving and interpreting signals in a time interval in the range of a few seconds to provide a card holder identified or card holder not identified output to allow or prohibit a transaction to proceed respectively.

Ideally, the time interval is approximately one second.

Preferably, the software control module has means for generating a unique bioelectrical impedance signature for a specific card holder from a full set of data including thumb-forefinger only loop, skin resistivity, sweat, geographical regionality, weight, age, gender, current, voltage measurement and frequency range.

Ideally, the identification card has means for transmitting live biometric data to an authorisation unit via freespace communications protocols.

Preferably, the identification card has means for transmitting an authorisation signal generated in response to successful identification of a cardholder to an authorisation unit via freespace communications protocols.

Ideally, an authorised card user's bioelectrical impedance signature is stored on the identification card in a read only memory (ROM).

Preferably, means for encrypting bioelectrical impedance signatures is stored on the memory of the controller.

Ideally, the identification card has an embedded radiation emitter for allowing the spatial location of the identification card to be defined at any given time.

Ideally, the software control module executes in response to the electrodes of the identification card being held between a card holder's finger and thumb for a predetermined period of time.

Preferably, a card holder's bioelectrical impedance signature is stored on the card in a read only memory, this signature being recorded during initial cardholder enrolment and primary data calibration in a secure environment.

Advantageously, the card does not require the information to be disclosed to or verified by any external comparative databases or reading applications, protecting the citizen's rights and removing the interoperability and infrastructural data protection issues.

Advantageously, the card reader does not require any additional sensing equipment.

Ideally, the software control module has error identifying means for identifying minor differences in biometric, sensor, presentation or transmission information which occur even though the person enrolled and the person currently operating the card are the same.

Advantageously, these error identifying means allow a transaction to proceed in event of minor differences.

Ideally, the error identifying means comprises robustly designed algorithms taking into account thumb-forefinger only loop, skin resistivity, sweat, geographical regionality, weight, age, gender, current, voltage measurement and frequency range.

Preferably, the card has means for indicating when a cardholder has been positively identified and when a cardholder has not been identified.

Ideally, the indicating means comprises a green and red L.E.D although the method of conveying the result of the verification phase can vary to accommodate the architecture and GUI on the card reader.

Advantageously, this alerts the cardholder to the fact that they have been successfully identified or not as the case may be and that they no longer need to hold the card between their forefinger and thumb allowing them to push the card fully into the slot in the card reader or to remove the card and try again.

Ideally, the identification cards are manufactured using injection moulding.

Preferably, the injection moulding techniques comprise double in mould labelled injection moulding using through-hole printing of conductive ink on thin film substrate layers and providing inter-connectivity across layers.

Preferably, the software control module has algorithms with means for generating a unique bioelectrical impedance signature for a specific card holder from a full set of data including thumb-forefinger only loop, skin resistivity, sweat, geographical regionality, weight, age, gender, current, voltage measurement and frequency range. The algorithms may combine the impedance with other biometric data as may be required (e.g. since the user is presenting finger and thumb on the card, those fingerprints may be captured and collated as part of a biometric signature).

Ideally, the algorithms are capable of reducing the time required to identify a live cardholder by utilising a subset of data selected from any combination of the above variables.

Preferably, the card is dimensioned to meet DIN EN 150 9002 namely 85.72 mm x 54.03 mm.

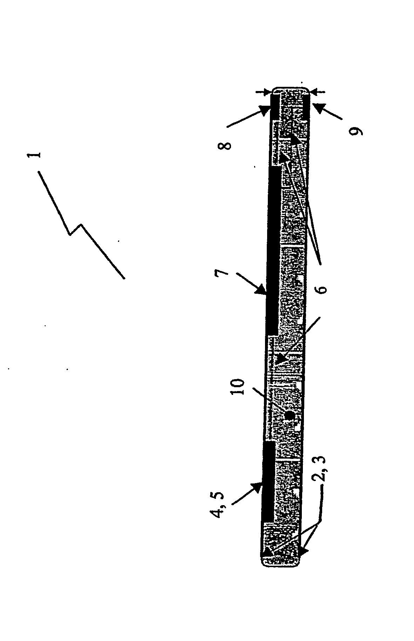

The invention will now be described with reference to the accompanying drawing which shows by way of example only one embodiment of an identification card in accordance with the invention.

Referring to the drawing there is shown a bioelectrical impedance identification card indicated generally by the reference numeral 1. The card 1 comprises a cover 2 of a thin film substrate layer 3 encapsulating a polymer layer 10. A C.P.U. 4 is located within the card 1 and has an externally contactable electrode 5. Electronic control circuitry 7 is in electrical communication with C.P.U. 4 via electrical connections 6 and is also in electrical communication with a pair of externally contactable electrodes 8, 9. The electrodes 8, 9 are disposed on opposed main surfaces of the cover 2 of thin film substrate layer 3. One electrode 8 is formed for receiving the thumb and the other electrode 9 is formed for receiving the forefinger. Of course, the electrodes 8, 9 can be formed identically so as to be suitable for receiving both the finger and the thumb.

In use, a card holder lifts the card 1 with their thumb on one electrode 8 and their forefinger on the other electrode 9. The card's own power source is initiated when the card holder holds the electrodes 8, 9 between their finger and thumb for a predetermined period of time. An operating system on the C.P.U. 4 boots up and a software control module is executed on the C.P.U. 4 generating and transmitting digital signals to the electronic control circuitry 7 which converts the digital signals to analogue current and frequency variable electrical signals and transmits these signals to the card holder's hand via electrodes 8, 9. The bioelectrical impedance of the card holder's body attenuates the signals as they pass around the closed loop formed by the thumb and forefinger located on the opposed electrodes 8, 9. When the electrodes 8, 9 receive the attenuated signals the signals are passed to the electronic control circuitry 7. The electronic control circuitry 7 reads voltage values for each frequency of the attenuated signals, filters the attenuated signals and converts them from analogue to digital and presents the signals to the software module running on the C.P.U. 4.

The software control module generates a bioelectrical impedance signature for the cardholder currently holding the card 1 from the information received from the electronic control circuitry 7. This bioelectrical impedance signature is then compared by the software control module with a bioelectrical impedance signature which has been recorded during a secure enrolment procedure for the entitled cardholder. If the signatures correlate to a degree determined to be sufficient by versatile comparison algorithms underlying a software comparison routine (intelligent agent) then an indicator on the card is powered and the card holder can proceed as normal with the transaction they wish to execute by inserting the card fully into the card reader slot or by transmitting the authorisation signal to a remote transaction unit. If the signatures do not correlate the cardholder can try again. If the identification process is unsuccessful after a number of attempts the cardholder is attempting to use a card which they are not authorised to use and a potentially fraudulent transaction has been prevented.

Variations and modifications can be made without departing from the scope of the invention as defined in the appended claims.

## Claims

1. An identification card (1) having means for generating and transmitting signals into the body of a card holder and means for receiving and interpreting the signals from the card holder's body, the signals being attenuated by bioelectrical impedance of the card holder's body such that the interpretation of the attenuated signals by the interpretation means provides a bioelectrical impedance signature for uniquely identifying the card holder, wherein the means for generating and transmitting signals into the body of a card holder and the means for receiving and interpreting the signals from the card holder's body comprises a controller having a central processing unit (C.P.U.) (4) and an associated memory, two or more electrodes (8,9) disposed on an external surface of the card and electronic control circuitry (7) electrically coupling the electrodes (8,9) to the controller, wherein the card has its own on board power source capable of providing sufficient power for independent operation of the identification card (1), **characterised in that** the two or more electrodes (8,9) are provided on opposing main faces of the identification card (1) and are located so that the forefinger and the thumb are naturally located thereon when the card is held between the forefinger and the thumb, and the card's own power source is initiated when the card holder holds the electrodes (8, 9) between their finger and thumb for a predetermined period of time.

2. An identification card (1) as claimed in claim 1, wherein the electronic control circuitry (7) comprises means for generating a range of analogue signals of varying currents and frequency.

3. An identification card (1) as claimed in claim 2, wherein the currents are in the range of 100µA to 900µA.

4. An identification card (1) as claimed in claim 2 or claim 3, wherein the frequency of the signals is in the range of 1KHZ to 1350 KHZ.

5. An identification card (1) as claimed in any one of the preceding claims, wherein the electronic control circuitry (7) has means for filtering the attenuated signals, converting the analogue signals to digital signals and passing the signals to the C.P.U. (4) for interpretation.

6. An identification card (1) as claimed in any one of preceding claims, wherein the means for generating and interpreting signals comprises a software control module stored on the memory of the controller.

7. An identification card (1) as claimed in claim 6, wherein the software control module compares a live bioelectrical impedance signature with a bioelectrical impedance signature recorded in the memory of the controller.

8. An identification card (1) as claimed in claim 6 or claim 7, wherein the software control module has means for identifying bioelectrical impedance characteristics representative of fat mass, body cell mass, extra cellular water and skeletal mass.

9. An identification card (1) as claimed in any one of claims 6 to 8, wherein the software control module is capable of generating, transmitting, receiving and interpreting signals in a time interval in the range of a few seconds to provide a cardholder identified or cardholder not identified output to allow or prohibit a transaction to proceed respectively.

10. An identification card (1) as claimed in any one of claims 6 to 9, wherein the software control module has means for generating a unique bioelectrical impedance signature for a specific card holder from a full set of data including thumb-forefinger only loop, skin resistivity, sweat, geographical regionality, weight, age, gender, current, voltage measurement and frequency range.

11. An identification card (1) as claimed in any one of the preceding claims, wherein the identification card (1) has means for transmitting live biometric data to an authorisation unit via freespace communications protocols.

12. An identification card (1) as claimed in any one of the preceding claims, wherein the identification card (1) has means for transmitting an authorisation signal generated in response to successful Identification of a cardholder to an authorisation unit via freespace communications protocols.

13. An identification card (1) as claimed in any one of the preceding claims, wherein an authorised card user's bioelectrical impedance signature is stored on the identification card (1) in a read only memory (ROM).

14. An identification card (1) as claimed in any one of the preceding claims, wherein means for encrypting bioelectrical impedance signatures is stored on the memory of the controller.

15. An identification card (1) as claimed in any one of the preceding claims, wherein the identification card (1) has an embedded radiation emitter for allowing the spatial location of the identification card (1) to be defined at any given time.

16. An identification card (1) as claimed in any one of claims 6 to 15, wherein the software control module executes in response to the electrodes (8,9) of the identification card (1) being held between a card holder's finger and thumb for a predetermined period of time.

## Patentansprüche

1. Eine Identifikationskarte (1) mit der Fähigkeit zur Erzeugung und Übertragung von Signalen in den Körper des Karteninhabers und der Fähigkeit zum Empfang und zur Interpretation der Signale vom Körper des Karteninhabers, bei der die Signale durch bioelektrische Impedanz des Körpers des Karteninhabers in der Weise gedämpft werden, dass die Interpretation der gedämpften Signale durch die Interpretationsmittel eine bioelektrische Impedanz-Signatur zur einzigartigen Identifizierung des Karteninhabers bietet, wobei die Fähigkeit zur Erzeugung und Übertragung von Signalen in den Körper eines Karteninhabers und die Fähigkeit zum Empfang und zur Interpretation der Signale vom Körper des Karteninhabers auf einem Regler mit einem zentralen Prozessor (C.P.U.) (4) und einem dazugehörigen Speicher, zwei oder mehr auf der Aussenseite der Karte angeordneten Elektroden (8,9) beruht und einem elektronischem Regelkreis (7) der die Elektroden (8,9) elektrisch mit dem Regler verkuppelt, wobei die Karte ihre eigene interne Stromquelle hat, die für den unabhängigen Betrieb der Identifikationskarte (1) genug Strom bietet, und **dadurch** charakterisiert ist, dass die zwei oder mehrere Elektroden (8,9) auf gegenüberliegenden Hauptseiten der Identifikationskarte (1) vorhanden und so angeordnet sind, dass Zeigefinger und Daumen natürlich aufliegen, wenn die Karte zwischen Zeigefinger und Daumen gehalten wird, und die Stromquelle des Karteninhabers initiiert wird, wenn der Karteninhaber die Elektroden (8,9) für die Dauer einer vorbestimmten Zeit zwischen Zeigefinger und Daumen hält.

2. Eine Identifikationskarte (1) gemäß Anspruch 1, bei der der elektronische Regelkreis (7) die Fähigkeit zur Erzeugung von einer Reihe von analogen Signalen verschiedener Stromstärken und Frequenzen besitzt.

3. Eine Identifikationskarte (1) gemäß Anspruch 2, bei der die Stromstärken zwischen 100µA und 900µA liegen..

4. Eine Identifikationskarte (1) gemäß Anspruch 2 oder 3, bei der die Frequenz der Signale zwischen 1 KHZ und 1350 KHZ liegt.

5. Eine Identifikationskarte (1) gemäß der vorhergehenden Ansprüche, bei der der elektronische Regelkreis (7) die Fähigkeit zum Filtern der gedämpften Signale besitzt und die analogen Signale in digitale Signale verwandelt und diese Signale zur Interpretation an den C.P.U. (4) weiterleitet.

6. Eine Identifikationskarte (1) gemäß der vorhergehenden Ansprüche, bei der die Fähigkeit zur Erstellung und Interpretation von Signalen auf einem auf dem Speicher des Reglers angeordneten Software Steuer-Modul beruht.

7. Eine Identifikationskarte (1) gemäß Anspruch 6, bei der das Software Steuer-Modul eine live Impedanz-Signatur mit einer auf dem Regler gespeicherten bioelektrischen Impedanz-Signatur vergleicht.

8. Eine Identifikationskarte (1) gemäß Anspruch 6 oder 7, bei der das Software Steuer-Modul die Fähigkeit zur Identifizierung von live bioelektrischen Impedanz-Charakteristika besitzt, die Fettmasse, Körperzellenmasse, extra-zelluläre Wassermasse und Skelettmasse darstellen.

9. Eine Identifikationskarte (1) gemäß der Ansprüche 6 bis 8, bei der das Software Steuer-Modul die Fähigkeit zur Erzeugung, Übertragung, zum Empfang und zur Interpretation von Signalen innnerhalb eines Zeitraumes von wenigen Sekunden zur Feststellung eines identifizierten oder nicht identifizierten Karteninhabers besitzt, um eine Transaktion zu unterbinden oder zu genehmigen.

10. Eine Identifikationskarte (1) gemäß der Ansprüche 6 bis 9, bei der das Software Steuer-Modul die Fähigkeit besitzt, für einen spezifischen Karteninhaber aus einem vollen Datensatz einschließlich von Daumen-Zeigefinger-Schleife, Hautwiderstandsfähigkeit, Schweiß, geografischer Regionalität, Gewicht, Alter, Geschlecht, Stromstärke, Volt-Messung und Frequenz eine einzigartige bioelektrische Impedanz-Signatur zu schaffen.

11. Eine Identifikationskarte (1) gemäß der vorhergehenden Ansprüche, bei der die Identifikationskarte (1) die Fähigkeit zur Übermittlung von live biometrischen Daten an eine Autorisierungsanlage über Free Space Kommunikations-Protokolle besitzt.

12. Eine Identifikationskarte (1) gemäß der vorhergehenden Ansprüche, bei der die Identifikationskarte (1) die Fähigkeit zur Übermittlung eines Autorisierungssignals in Beantwortung einer erfolgreichen Identifizierung eines Karteninhabers an eine Autorisierungsanlage über Free Space Kommunikations-Protokolle besitzt.

13. Eine Identifikationskarte (1) gemäß der vorhergehenden Ansprüche, bei der die bioelektrische Impedanz-Signatur eines autorisierten Karteninhabers auf der Identifkationskarte (1) als Read Only Memory (ROM) gespeichert wird.

14. Eine Identifikationskarte (1) gemäß der vorhergehenden Ansprüche, bei der die Fähigkeit zur Verschlüsselung von bioelektrischen Impedanz-Signaturen auf dem Speicher des Reglers gespeichert wird.

15. Eine Identifikationskarte (1) gemäß der vorhergehenden Ansprüche, bei der die Identifikationskarte (1) einen eingebauten Strahler besitzt, der zu jeder Zeit eine räumliche Lokalisierung der Identifizierungskarte (1) ermöglicht.

16. Eine Identifikationskarte (1) gemäß der Ansprüche 6 bis 15, bei der das Software Steuer-Modul in Beantwortung der Elektroden (8,9) der für einen vorbestimmten Zeitraum zwischen Finger und Daumen des Karteninhabers gehaltenen Identifikationskarte (1) eine Leistung ausführt.

## Revendications

1. Une carte d'identification (1) comportant des moyens pour produire et emettre des signaux jusque dans l'intérieur du corps d'un porteur de carte, et des moyens de recevoir et d'interpréter les signaux provenant du corps du porteur de carte, les signaux étant atténués par impédance bioélectrique du corps du porteur de carte de façon à ce que l'interprétation des signaux atténués par le moyen d'interprétation fournisse une signature à impédance bioélectrique permettant d'identifier de manière non équivoque le porteur de carte, par lequel le moyen pour produire et émettre les signaux dans le corps d'un porteur de carte et le moyen de recevoir et d'interpréter les signaux provenant du corps du porteur de carte se compose d'un contrôleur muni d'un processeur central (P.C.)(4) et d'une mémoire y étant associée, deux électrodes (8, 9) ou plus, placés sur la surface externe de la carte, et de circuits de régulation électronique (7) raccordant les électrodes (8, 9) de manière électrique au contrôleur par lequel la carte est munie de sa propre source d'énergie intégrée pouvant fournir suffisamment d'énergie pour le fonctionnement autonome de la carte d'identification (1), **caracterisé par le fait que** deux électrodes (8, 9) ou plus sont prévus sur les principales faces opposées de la carte d'identification (1) et sont situés de manière à ce que l'index et le pouce se placent naturellement dessus lorsque la carte est maintenue entre l'index et le pouce, et que la propre source d'énergie de la carte soit amorcée quand le porteur de la carte tient les électrodes (8, 9) entre son doigt et son pouce pendant une période de temps prédéterminée.

2. Une carte d'identification (1) comme revendiqué dans la Revendication 1, par laquelle les circuits de régulation électronique (7) comportent le moyen de produire une fourchette de signaux analogues de courants et fréquence variées.

3. Une carte d'identification (1) comme revendiqué dans la Revendication 2, par laquelle les courants sont compris dans une fourchette de 100 à 900µA.

4. Une carte d'identification (1) comme revendiqué dans les Revendications 2 ou 3, par laquelle la fréquence des signaux se trouve dans une fourchette de 1KHZ à 1350KHZ.

5. Une carte d'identification (1) comme revendiqué dans l'une ou l'autre des revendications précédentes, par laquelle les circuits de régulation électronique (7) ont le moyen de filtrer les signaux attenués, et de convertir les signaux analogues en signaux numériques et d'émettre les signaux au P.C (4) pour qu'ils soient interprétés.

6. Une carte d'identification (1) comme revendiqué dans l'une ou l'autre des revendications précédentes, par laquelle le moyen de produire et d'interpréter les signaux comprennent un module de commande de logiciel stocké dans la mémoire du contrôleur.

7. Une carte d'identification (1) comme revendiqué dans la Revendication 6, par laquelle le module de commande de logiciel compare une signature à impédance bioélectrique en temps réel avec une signature à impédance bioélectrique enregistrée dans la mémoire du contrôleur.

8. Une carte d'identifcation (1) comme revendiqué dans la Revendication 6 ou 7, par laquelle le module de commande de logiciel a le moyen d'identifier les caractéristiques d'impédance bioélectrique représentatives de la masse adipeuse, masse des cellules corporelles, masse de l'eau extra-cellulaire et de la masse skelettique.

9. Une carte d'identification (1) comme revendiqué dans l'une ou l'autre des revendications 6 a 8, par laquelle le module de commande de logiciel est en mesure de produire, émettre, recevoir et interpréter des signaux dans un intervalle de temps compris dans une fourchette de quelques secondes pour fournir un résultat indiquant que le porteur de carte est identifié ou que le porteur de carte est non-identifié et permettre ou interdir qu'une transaction soit complétée respectivement.

10. Une carte d'identification (1) comme revendiqué dans l'une ou l'autre des revendications 6 à 9, par laquelle le module de commande de logiciel ait le moyen de produire une signature unique à impédance bioélectrique pour un porteur de carte particulier à partir d'un ensemble complet de données telles que la boucle unique pouce-index, résistivité de la peau, sueur, région géographique, poids, âge, genre, courant, la mesure du courant et la fourchette de fréquence.

11. Une carte d'identification (1) comme revendiqué dans l'une ou l'autre des revendications précédentes, par laquelle la carte d'identification (1) a le moyen d'émettre des données biométriques en temps réel vers une unité d'autorisation par l'intermédiaire de protocoles de communications.

12. Une carte d'identification (1) comme revendiqué dans l'une ou l'autre des revendications précédentes, par laquelle la carte d'identification a le moyen d'émettre un signal d'autorisation généré en réponse à l'identification positive d'un porteur de cartes envoyé à une unité d'autorisation par l'intermédiaire de protocoles de communication.

13. Une carte d'identifcation (1) comme revendiqué dans l'une ou l'autre des revendications précédentes, par laquelle la signature à impédance bioélectrique d'un utilisateur de carte autorisé est mémorisée dans la mémoire morte de la carte d'identification (1).

14. Une carte d'identification (1) comme revendiqué dans l'une ou l'autre des revendications précédentes, par laquelle des moyens d'encrypter les signatures à impédance bioélectrique sont stockés dans la mémoire du contrôleur.

15. Une carte d'identification (1) comme revendiqué dans l'une ou l'autre des revendications précédentes, par laquelle la carte d'identification (1) est munie d'un émetteur de radiation intégré pour permettre de définir la localisation spatiale de la carte d'identification (1) à tout moment donné.

16. Une carte d'identification (1) comme revendiqué dans l'une ou l'autre des revendications 6 à 15, par laquelle le module de commande de logiciel fonctionne en réponse aux électrodes (8, 9) de la carte d'identification (1) étant maintenue entre le doigt et le pouce du porteur de carte pendant une période de temps prédéterminée.
